(19) Europäisches Patentamt
European Patent Office
Office européen des brevets

(11) EP 1 266 904 B1

(12) **EUROPÄISCHE PATENTSCHRIFT**

(45) Veröffentlichungstag und Bekanntmachung des Hinweises auf die Patenterteilung:
**13.09.2006 Patentblatt 2006/37**

(51) Int Cl.:
*C07F 9/06* (2006.01) *C07C 17/20* (2006.01)
*B01J 31/02* (2006.01)

(21) Anmeldenummer: **02011682.8**

(22) Anmeldetag: **03.06.2002**

(54) **Verbessertes Verfahren zur Herstellung kernfluorierter Aromaten**

Improved process for preparing fluorinated aromatic compounds

Procédure améliorée pour la préparation des composés aromatiques fluorés

(84) Benannte Vertragsstaaten:
**CH DE FR GB IT LI**

(30) Priorität: **15.06.2001 DE 10129057**

(43) Veröffentlichungstag der Anmeldung:
**18.12.2002 Patentblatt 2002/51**

(73) Patentinhaber: **Saltigo GmbH**
**51369 Leverkusen (DE)**

(72) Erfinder:
- **Henrich, Marielouise, Dr.**
  **51373 Leverkusen (DE)**
- **Marhold, Albrecht, Dr.**
  **51373 Leverkusen (DE)**
- **Kolomeitsev, Alexander, Dr.**
  **01133 Kiew (UA)**
- **Röschenthaler, Gerd-Volker, Prof. Dr.**
  **28357 Bremen (DE)**

(74) Vertreter: **Wichmann, Birgid et al**
**LANXESS Deutschland GmbH**
**LIP IPR**
**Q 18**
**51369 Leverkusen (DE)**

(56) Entgegenhaltungen:
**EP-A- 0 691 334**
**EP-A- 0 762 202**
**EP-A- 0 791 600**
**WO-A-87/04149**
**WO-A-98/05610**
**DE-A- 19 702 282**
**US-A- 4 287 374**

- **SCHWESINGER R ET AL: "Stable Phosphazenium Ions in Synthesis - an Easily Accessible, Extremely Reactive "Naked" Fluoride Salt" ANGEWANDTE CHEMIE, INTERNATIONAL EDITION, VERLAG CHEMIE. WEINHEIM, DE, Bd. 30, Nr. 10, 1991, Seiten 1372-1375, XP002941731 ISSN: 0570-0833**
- **DORTA ET AL: "The [IrCl(Diphosphine)] 2/Fluoride System. Developing Catalytic Asymmetric Olefin Hydroamination" JOURNAL OF THE AMERICAN CHEMICAL SOCIETY, AMERICAN CHEMICAL SOCIETY, WASHINGTON, DC, US, Bd. 119, Nr. 44, 5. November 1997 (1997-11-05), Seiten 10857-10858, XP002110586 ISSN: 0002-7863**
- **SCHWESINGER R ET AL: "Extremely strong, uncharged auxiliary bases; monomeric and polymer-supported polyaminophosphazenes (P2-P5)" LIEBIGS ANNALEN, 1996, Seiten 1055-1081, XP002139767 ISSN: 0947-3440**
- **HAMED A ET AL: "2-Azoniaallene Salts From the Reaction of Chlorocarbenium Salts with N-Silylimines" SYNTHESIS, Nr. 5, 1989, Seiten 400-2, XP001083979 ISSN: 0039-7881**
- **MARCHENKO A ET AL: "N'''-Chlorophosphorimidic triamides" JOURNAL OF GENERAL CHEMISTRY OF THE USSR (ENGLISH TRANSLATION), Bd. 53, Nr. 3, 1983, Seiten 583-9, XP001087779 ISSN: 0022-1279**

EP 1 266 904 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein verbessertes Verfahren zur Herstellung kernfluorierter Aromaten durch eine Halogenaustauschreaktion (Halex-Reaktion) in Gegenwart eines Katalysators.

Kernfluorierte Aromaten sind wichtige Zwischenprodukte zur Herstellung von biologisch aktiven Substanzen für pharmazeutische und agrochemische Anwendungen.

Es ist bekannt, Halex-Reaktionen in aprotischen, stark polaren Lösungsmitteln unter Einsatz von Metallfluoriden bei erhöhter Temperatur und in Gegenwart von Alkylammonium- oder Alkylphosphonium-Salzen (US-A 4 287 374), Pyridiniumsalzen (WO 87/04149), Kronenethern (DE-A 197 02 282) oder Tetraamidophosphoniumsalzen (WO 98/05610) durchzuführen. Nachteilig bei derartigen Reaktionen sind, insbesondere beim Einsatz schwach aktivierter Aromaten, die benötigten hohen Reaktionstemperaturen und langen Reaktionszeiten. Dies führt zu hohen Energieverbräuchen und niedrigen Raum-Zeit-Ausbeuten. Die hohen Reaktionstemperaturen führen häufig zur Bildung von unerwünschten Neben- und Zersetzungsprodukten. Außerdem benötigt man große Mengen teurer Lösungsmittel.

Weiterhin sind z.B. die Tetraamidophosphoniumsalze (WO 98/05610) außerordentlich toxisch.

Es besteht deshalb noch das Bedürfnis nach einem Verfahren zur Herstellung von kernfluorierten Aromaten durch eine Halex-Reaktion, bei dem weniger Energie verbraucht wird, höhere chemische und Raum-Zeit-Ausbeuten möglich sind und gegebenenfalls auf Lösungsmittel verzichtet werden kann.

Es wurde nun ein Verfahren zur Herstellung von kernfluorierten Aromaten durch Umsetzung einer aromatischen Verbindung, die am Kern mit gegen Fluor austauschbarem Halogen substituiert ist, mit einem Fluorid, gefunden, das dadurch gekennzeichnet ist, dass man es bei 40 bis 260°C und in Gegenwart mindestens einer Verbindung der Formel (I) durchführt

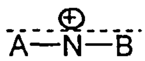 $An^{\ominus}$ (I),

in der

A für einen Rest der Formeln (II) oder (III)

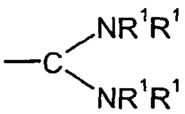

$-P(NR^1R^1)_3$

(II) (III)

und

B unabhängig von A für einen Rest der Formeln (II), (III), (IV) oder (IVa)

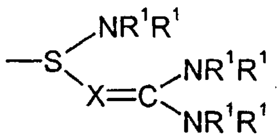

(IV)

$-S(NR^1R^1)_2$ (IVa)

stehen,
wobei
die einzelnen $R^1$ gleich oder verschieden sind und jeweils für geradkettiges oder verzweigtes $C_1$-$C_{10}$-Alkyl,

geradkettiges oder verzweigtes $C_2$-$C_{10}$-Alkylen oder $C_6$-$C_{12}$-Aryl stehen,
wobei eine oder mehrere $NR^1R^1$-Gruppen auch für einen 3- bis 5-gliedrigen, gesättigten oder ungesättigten Ring stehen können, der aus einem Stickstoffatom und sonst C-Atomen gebildet wird,
wobei die Formel (II) und die

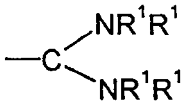

-Gruppe in Formel (IV) auch für den Rest eines gesättigten oder ungesättigten 4- bis 8-gliedrigen Rings stehen kann, der zwei Stickstoffatome und sonst Kohlenstoffatome enthält,

X für Stickstoff oder Phosphor steht und

$An^{\ominus}$ ein Äquivalent eines Anions bedeutet.

Vorzugsweise sind die beiden an das gleiche Stickstoffatom gebundenen $R^1$-Reste gleich.

Vorzugsweise stehen die Reste $R^1$ für Methyl, Ethyl, Propyl oder Butyl oder eine $NR^1R^1$-Gruppe für einen 5- bis 7-gliedrigen gesättigten oder ungesättigten Ring, der aus einem Stickstoffatom und sonst C-Atomen gebildet wird oder die Formel (II) oder die

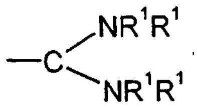

-Gruppe in Formel (IV) für einen gesättigten 5- bis 7-gliedrigen Ring, der 2 Stickstoffatome und sonst Kohlenstoffatome enthält,

X X für Stickstoff und

$An^{\ominus}$ für Chlorid, Bromid, $(CH_3)_3SiF_2^{\ominus}$, $HF_2^{\ominus}$, $H_2F_2^{\ominus}$, Tetrafluoroborat, Hexafluorophosphat, Carbonat oder Sulfat.

Ganz besonders bevorzugte Verbindungen der Formel (I) sind solche, die den Formeln (V) bis (IX) entsprechen.

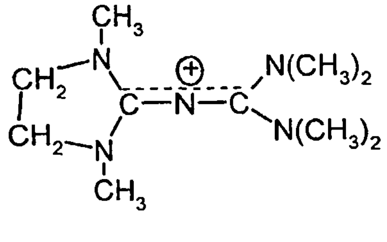 $Cl^{\ominus}$ (V)

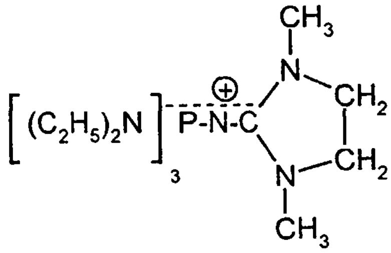 $Cl^{\ominus}$ (VI)

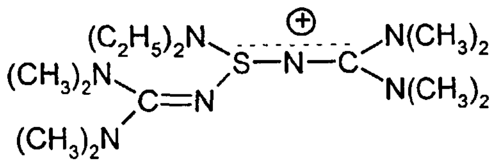

Br$^{\ominus}$ (VII)

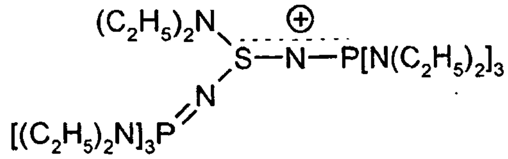

Br$^{\ominus}$ (VIII)

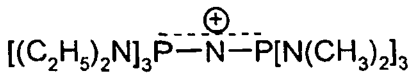

Br$^{\ominus}$ (IX)

Es ist ein Vorteil des erfindungsgemäßen Verfahrens, dass man es auf eine große Anzahl von aromatischen Verbindungen anwenden kann, die mit gegen Fluor austauschbarem Halogen substituiert sind, und so eine große Anzahl von kernfluorierten Aromaten herstellen kann. Bei dem Halogen, das gegen Fluor austauschbar ist, kann es sich z.B. um Chlor und/oder Brom handeln. Bevorzugt dabei ist Chlor.

Beispielsweise kann man mit dem erfindungsgemäßen Verfahren in vorteilhafter Weise kernfluorierte Aromaten der Formel (X) herstellen

$$R^2{}_xArF_wCl_{(y-w)}R_z{}^3 \qquad (X),$$

in der

- $R^2$ unabhängig voneinander für F, Cl, Br, $NO_2$, CN, $CF_3$, $CCl_3$, CHO, $OCF_3$, $SCF_3$, $COR^4$, $COOR^4$, COY oder $SO_2Y$ mit $R^4$ = $C_1$-$C_{10}$-Alkyl und Y = F, Cl, Br oder $CF_3$ steht,
- $R^3$ unabhängig voneinander für Wasserstoff oder einen geradkettigen oder verzweigten $C_1$-$C_{10}$-Alkyl- oder $C_1$-$C_{10}$-Alkoxyrest steht,
- x eine ganze Zahl von 1 bis 3 bedeutet,
- Ar für einen aromatischen oder heteroaromatischen Rest mit insgesamt 6 bis 10 Ringatomen steht, wobei es sich bei den Ringatomen nur um Kohlenstoffatome handeln kann, aber auch um Kohlenstoffatome plus 1 bis 3 Heteroatome aus der Gruppe Stickstoff, Sauerstoff und Schwefel, steht,
- w eine ganze Zahl von 1 bis y bedeutet,
- y eine ganze Zahl von 1 bis 5 bedeutet und
- z Null oder eine ganze Zahl von 1 bis 5 bedeutet, wobei gilt: x+y+z = Zahl aller substituierbaren Valenzen am Rest Ar.

Bevorzugt können als aromatische Verbindungen, die mit gegen Fluor austauschbarem Halogen substituiert sind, solche eingesetzt werden, die der Formel (XI) entsprechen

$R^2_xArCl_yR^3_z$ (XI),

wobei die verwendeten Symbole die bei Formel (X) angegebene Bedeutung haben.

Vorzugsweise stehen in den Formeln (X) und (XI)

$R^2$ unabhängig voneinander für Cl, $NO_2$, CN, $CF_3$, COCl oder CHO,

$R^3$ unabhängig voneinander für Wasserstoff, Methyl, Ethyl, Methoxy oder Ethoxy,

Ar für einen Phenyl- oder Pyridylrest,

x für 1 oder 2,

w für 1 oder 2,

y für eine ganze Zahl von 1 bis 4 und

z für Null oder 1.

Beispielhafte Verbindungen der Formel (XI) sind: 2,3,4,5-Tetrachlorbenzotrifluorid, 4-Chlornitrobenzol, 3,4-Dichlorbenzonitril, 2,6-Dichlorbenzonitril, 2,4-Dichlorbenzaldehyd, 3,4,5-Trichlorpyridin, 4-Chlorbenzaldehyd, 3,4-Dichlorbenzotrifluorid, 1,2,3-Trichlorbenzol und 2,6-Dichlorbenzoylchlorid.

Als Fluoride zum Austausch von Halogen gegen Fluor kommen beispielsweise Alkali-, Erdalkali- und Ammoniumfluoride in Frage. Bevorzugt sind Kaliumfluorid, Natriumfluorid, Calciumfluorid und Ammoniumfluorid sowie deren Gemische untereinander sowie deren Gemische mit Lithium-, Rubidium- und/oder Cäsiumfluorid.

Bezogen auf 1 Mol gegen Fluor auszutauschendes Halogen, das an den Kern einer aromatischen Verbindung gebunden ist, kann man beispielsweise 0,001 bis 0,5 Mole, vorzugsweise 0,01 bis 0,02 Mole einer oder mehrerer Verbindungen der Formel (I) und beispielsweise 0,8 bis 2 Äquivalente, vorzugsweise 1,1 bis 1,3 Äquivalente eines oder mehrerer Fluoride einsetzen.

Das erfindungsgemäße Verfahren wird vorzugsweise bei Temperaturen im Bereich 70 bis 220°C durchgeführt. Besonders bevorzugt sind 90 bis 200°C.

Man kann das erfindungsgemäße Verfahren in Anwesenheit oder in Abwesenheit von Lösungsmitteln durchführen. Insbesondere bei der Umsetzung von polychlorierten Benzotrifluoriden zu fluorierten und chlorierten Benzotrifluoriden und/oder zu polyfluorierten Benzotrifluoriden kann auf ein Lösungsmittel verzichtet werden. Wenn man Lösungsmittel einsetzen möchte, kommen beispielsweise dipolar aprotische und unpolar aprotische Lösungsmittel in Frage. Geeignete dipolar aprotische Lösungsmittel sind beispielsweise Dimethylsulfoxid, Sulfolan, Dimethylformamid, Dimethylacetamid, 1,3-Dimethylimidazolin-2-on, N-Methylpyrrolidon, Acetonitril und Benzonitril. Geeignete unpolare aprotische Lösungsmittel sind beispielsweise Benzol, Toluol, Chlorbenzol, Dichlorbenzole, Chlortoluole und Chloralkane wie Dichlormethan.

Unpolar aprotische und dipolar aprotische Lösungsmittel kann man in beliebigen Mengen einsetzen, beispielsweise in Mengen von 0,1 bis 500 Gew.-%, vorzugsweise in Mengen von 0,2 bis 40 Gew.-%, jeweils bezogen auf die eingesetzte aromatische Verbindung, die mit gegen Fluor austauschbarem Halogen substituiert ist.

Man kann auch Gemische von Lösungsmitteln einsetzen, wobei es bevorzugt ist solche Lösungsmittelgemische einzusetzen, die 50 Gew.-% oder mehr dipolar aprotische Lösungsmittel enthalten.

Die Reaktionszeit beim erfindungsgemäßen Verfahren kann beispielsweise im Bereich von 2 bis 36 Stunden liegen.

Das erfindungsgemäße Verfahren kann bei vermindertem, normalem oder erhöhtem Druck durchgeführt werden. Vorzugsweise arbeitet man bei Normaldruck oder schwach erhöhtem Druck, z.B. bei 1 bis 6 bar.

Grundsätzlich können die Verbindungen der Formel (I) in Anwesenheit oder Abwesenheit von Luftsauerstoff gehandhabt werden. Es ist jedoch bevorzugt, die Verbindungen der Formel (I) unter Schutzgas zu handhaben und das erfindungsgemäße Verfahren unter Schutzgas durchzuführen. Geeignete Schutzgase sind beispielsweise Stickstoff und Argon.

Das erfindungsgemäße Verfahren lässt sich diskontinuierlich oder kontinuierlich durchführen.

Zur Aufarbeitung des nach Durchführung des erfindungsgemäßen Verfahren vorliegenden Reaktionsgemisches kann man beispielsweise so verfahren, dass man das Reaktionsgemisch nach Abkühlung mit Wasser mischt, die sich bildende organische Phase abtrennt und die abgetrennte organische Phase bei reduziertem Druck fraktioniert destilliert. Man kann das nach Durchführung des erfindungsgemäßen Verfahrens vorliegende Reaktionsgemisch auch direkt einer Destillation unterwerfen.

Ferner kann man dem Reaktionsgemisch ein Lösungsmittel zusetzen, feste Bestandteile durch Filtration abtrennen und das Filtrat bei reduziertem Druck destillieren. Es sind auch andere Aufarbeitungsmöglichkeiten anwendbar.
Die Verbindungen der Formel (I), bei denen A und B gleich sind und jeweils einem Rest der Formeln (II) oder (III) entsprechen, sind auf bekannte Weise oder analog dazu herstellbar (siehe Synthesis 1979, 215-216 und Angewandte Chemie 104, 864, 1992)).
Die vorliegende Erfindung betrifft auch ein Verfahren zur Herstellung von beliebigen Verbindungen der Formel (I), das dadurch gekennzeichnet ist, dass man eine Verbindung der Formel

$$[A\text{-}An']^{\oplus} \quad An^{\ominus} \qquad (XII),$$

in der

A die bei Formel (I) angegebene Bedeutung hat oder der Formel (IVa) entspricht,

An' für Chlor oder Brom und

$An^{\ominus}$ für ein Äquivalent eines Anions steht,

mit einer Verbindung der Formel (XIII)

$$HN{=}A' \qquad (XIII),$$

in der

A' hinsichtlich der Anordnung der Atome die bei Formel (I) für A angegebene Bedeutung hat, aber 2-bindig ist

umsetzt und eine Base zufügt.
Ein Beispiel für eine derartige Umsetzung ist

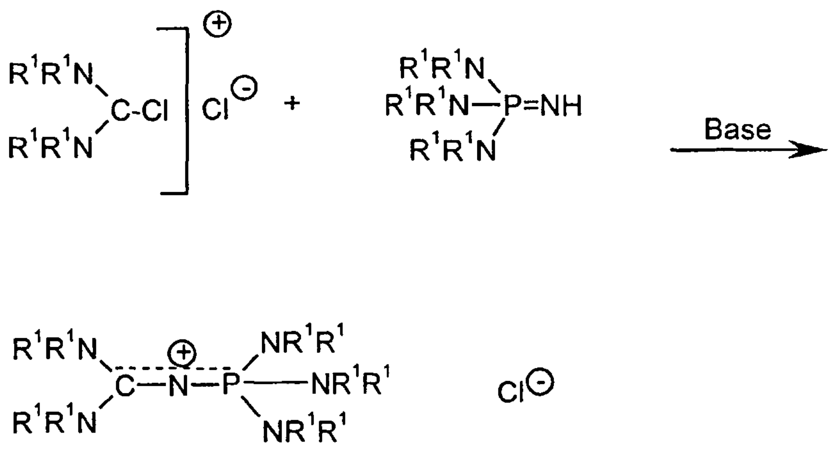


In den Formeln (XII) und (XIII) haben die verwendeten Symbole bevorzugt die Bedeutungen, die bei Formel (I) als bevorzugt angegeben ist.
Als Basen kommen beispielsweise Alkoholate, tertiäre Amine und im Überschuss eingesetzte Verbindungen der Formel (XIII) in Frage. Bevorzugt sind Natrium- und Kaliumalkoholate von geradkettigen oder verzweigten $C_1$- bis $C_4$-Alkylalkoholen und Tri-$C_1$-$C_{10}$-alkylamine. Besonders bevorzugt sind Natriummethylat, Natriumethylat und Triethylamin. Wenn Verbindungen der Formel (XIII) einigermaßen kostengünstig zur Verfügung stehen, beispielsweise ist das bei Tetraalkylguanidin der Fall, dann sind auch Überschüsse an Verbindungen der Formel (XIII) als Basen besonders bevorzugt.
Verbindungen der Formel (XII) kann man auf bekannte Weise oder analog dazu herstellen, z.B. durch Halogenierung des entsprechenden Harnstoffs, beispielsweise mit $SOCl_2$, $(COCl)_2$ oder $COCl_2$ oder durch Umsetzung von Phosphorpentachlorid mit einem sekundären Amin wie Diethylamin oder durch Halogenierung einer Verbindung des

Typs $(R_2N)_2S$ mit beispielsweise Brom.

Verbindungen der Formel (XIII) kann man auch auf bekannte Weise oder analog dazu herstellen, z.B. durch Umsetzung von Phosphorpentachlorid mit einem sekundären Amin, Ammoniak und einer Alkalilauge z.B. gemäß folgender Gleichung:

$$PCl_5 + Et_2NH \rightarrow (Et_2N)_3^{\oplus}P\text{-}Cl\ Cl^{\ominus} \xrightarrow[NH_3]{} (Et_2N)_3^{\oplus}P\text{-}NH_2\ Cl^{\ominus} \xrightarrow[NaOH]{} (Et_2N)_3P{=}NH$$

Et = Ethyl

Die Verbindungen der Formel (XIII) können auch in Form ihrer Hydrohalogenide eingesetzt werden, wie sie häufig bei ihrer Herstellung zunächst anfallen.

Beim erfindungsgemäßen Verfahren zur Herstellung von Verbindungen der Formel (I) kann man eine Verbindung der Formel (XIII) beispielsweise in einer Menge von 0,8 bis 3 Mol, vorzugsweise 1 bis 2 Mol, bezogen auf die Verbindung der Formel (XII), einsetzen.

Die Umsetzung der Verbindungen der Formeln (XII) und (XIII) kann beispielsweise bei Temperaturen im Bereich -80 bis +70°C, vorzugsweise im Bereich -70 bis +20°C durchgeführt werden. Innerhalb dieser Temperaturbereiche ist es vorteilhaft, für die Knüpfung von N-C-Bindungen bei relativ höheren, für die Knüpfung von N-P-Bindungen bei mittleren und für die Knüpfung von N-S-Bindungen bei relativ niedrigeren Temperaturen zu arbeiten.

Als Lösungsmittel kommen beispielsweise chlorierte aliphatische und aromatische Kohlenwasserstoffe, Ether, insbesondere cyclische Ether, Nitrile, Amide, Sulfoxide und aliphatische und aromatische Kohlenwasserstoffe in Frage. Bevorzugt sind Methylenchlorid, 1,2-Dichlorethan, Tetrahydrofuran, Dioxan, Toluol, Acetonitril, Dimethylformamid und Dimethylsulfoxid. Bei der Auswahl des Lösungsmittel ist darauf zu achten. dass es unter Reaktionsbedingungen nicht in den festen Zustand übergeht.

Die Umsetzung einer Verbindung der Formel (XII) mit einer Verbindung der Formel (XIII) ist im allgemeinen nach 0,5 bis 24 Stunden, häufig nach 4 bis 12 Stunden beendet. Dann kann dann die Base, z.B. in einer Menge von 1 bis 1,2 Äquivalenten, bezogen auf ein Mol der Verbindung (XII), zugeführt werden. Wenn man als Base einen Überschuss der Verbindung (XIII) einsetzt, dann kann man z.B. insgesamt 2 bis 2,2 Mole der Verbindung der Formel (XIII), bezogen auf die Verbindung (XII) einsetzen. Als Lösungsmittel für Alkoholate und tertiäre Amine sind insbesondere Alkohole geeignet. Überschüssige Verbindungen der Formel (XIII) benötigen kein zusätzliches Lösungsmittel. Die Zugabe der Base kann beispielsweise bei -50 bis +40°C, vorzugsweise bei -10 bis +10°C erfolgen. Es ist vorteilhaft, das Reaktionsgemisch nach Beendigung der Basenzugabe noch einige Zeit, beispielsweise 0,5 bis 1 Stunde, im angegebenen Temperaturbereich nachzurühren.

Zur Aufarbeitung des Reaktionsgemisches kann man z.B. nach Abtrennung der festen Bestandteile Lösungsmittel abziehen und das dann vorliegende Produkt z.B. durch Ausrühren mit einem Lösungsmittel, beispielsweise einem Keton, Ether oder Kohlenwasserstoff, reinigen. Es sind auch andere Aufarbeitungsmöglichkeiten denkbar.

Es ist vorteilhaft, die Synthese und Aufarbeitung von Verbindungen der Formel (I) unter einer Schutzgasatmosphäre durchzuführen, z.B. unter Stickstoff oder Argon.

Die erfindungsgemäß hergestellten Verbindungen der Formel (I) sind so wie sie nach der oben beschriebenen Aufarbeitung erhalten werden zum Einsatz als Katalysator für Halexreaktionen geeignet. In einigen Fällen ist beobachtet worden, dass bei der erfindungsgemäßen Herstellung von Verbindungen der Formel (I) Gemische von zwei oder mehr Einzelverbindungen anfallen, die der Formel (I) entsprechen. Auch solche Stoffgemische sind als Katalysatoren für Halexreaktionen geeignet. Bei diesen Stoffgemische kann es sich z.B. um solche handeln, die Verbindungen der Formel (I) mit A = Formel (II) und B = Formel (IV) und Formel (IVa) oder die Verbindungen der Formel (I) mit A = Formel (III) und B = Formel (IV) und Formel (IVa) enthalten. Mit dem erfindungsgemäßen Verfahren zur Herstellung von Verbindungen der Formel (I) wird ein universell anwendbares Verfahren zur Verfügung gestellt, mit dem Verbindungen der Formel (I) auf einfache und effiziente Weise zugänglich sind.

Die Erfindung betrifft weiterhin Verbindungen der Formel (I)

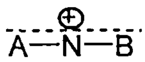 An⊖ (I),

in der

A für einen Rest der Formeln (II) oder (III)

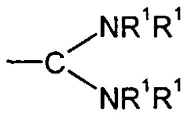

(II)

$-P(NR^1R^1)_3$

(III)

und

B unabhängig von A für einen Rest der Formeln (IV) oder (IVa)

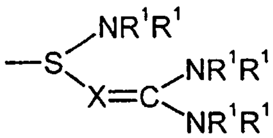

(IV)

$-S(NR^1R^1)_2$ (IVa)

stehen,

wobei
die einzelnen $R^1$ gleich oder verschieden sind und jeweils für geradkettiges oder verzweigtes $C_1$-$C_{10}$-Alkyl, geradkettiges oder verzweigtes $C_2$-$C_{10}$-Alkylen oder $C_6$-$C_{12}$-Aryl stehen,
wobei eine oder mehrere $NR^1R^1$-Gruppen auch für einen 3- bis 5-gliedrigen, gesättigten oder ungesättigten Ring stehen können, der aus einem Stickstoffatom und sonst C-Atomen gebildet wird,
wobei die Formel (II) und die

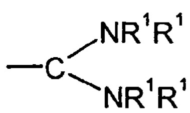

-Gruppe in Formel (IV) auch für den Rest eines gesättigten oder ungesättigten 4- bis 8-gliedrigen Rings stehen kann, der zwei Stickstoffatome und sonst Kohlenstoffatome enthält,

X für Stickstoff oder Phosphor steht und

An⊖ ein Äquivalent eines Anions bedeutet.

Vorzugsweise stehen die Reste $R^1$ für Methyl, Ethyl, Propyl oder Butyl oder eine $NR^1R^1$-Gruppe für einen 5- bis 7-gliedrigen gesättigten oder ungesättigten Ring, der aus einem Stickstoffatom und sonst C-Atomen gebildet wird oder

die Formel (II) oder die

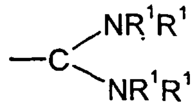

-Gruppe in Formel (IV) für einen gesättigten 5- bis 7-gliedrigen Ring, der 2 Stickstoffatome und sonst Kohlenstoffatome enthält,

X für Stickstoff und

$An^{\ominus}$ für Chlorid, Bromid, $(CH_3)_3SiF_2^{\ominus}$, $HF_2^{\ominus}$, $H_2F_2^{\ominus}$, Tetrafluoroborat, Hexafluorophosphat, Carbonat oder Sulfat.

Besonders bevorzugt sind Diethylamino-bis-(tetramethylguanidino)-sulfoniumbromid [Formel (VII)] und Diethylamino-bis-[tris-(diethylamino)-phosphazenyl]-sulfoniumbromid [Formel(VIII)].

Diese erfindungsgemäßen Verbindungen sind, wie beschrieben, über das erfindungsgemäße Herstellungsverfahren zugänglich.

Das erfindungsgemäße Verfahren zur Herstellung von kernfluorierten Aromaten verwendet effektivere Katalysatoren, erfordert weniger Energie, ermöglicht höhere chemische und Raum-Zeit-Ausbeuten und kann gegebenenfalls ohne Lösungsmittelzusatz durchgeführt werden. Häufig sind bei diesem erfindungsgemäßen Verfahren weniger toxische Verbindungen zu handhaben als bei den Verfahren des Standes der Technik. Selbst, wenn in einem konkreten Einzelfall nur ein oder zwei dieser Vorteile realisierbar sein sollten, handelt es sich um ein gegenüber dem Stand der Technik deutlich verbessertes Verfahren.

**Beispiele**

**Beispiel 1** (erfindungsgemäße Herstellung von 4-Nitrofluorbenzol)

In einem 1 1-Vierhalskolben, der mit Thermometer, Ankerrührer und Rückflusskühler mit Blasenzähler ausgerüstet war, wurden 157 g 4-Nitrochlorbenzol, 200 g Dimethylsulfoxid, 62,7 g Kaliumfluorid und 2,49 g (N,N-Dimethylimidazolidino)-tetramethylguanidiniumchlorid vorgelegt. Das Gemisch wurde unter Rühren auf 170°C erhitzt und 5 Stunden bei dieser Temperatur gehalten. Danach wurde das Reaktionsgemisch auf Raumtemperatur abgekühlt, Wasser im Volumenverhältnis 1:1 zugegeben, die sich bildenden Phasen getrennt und aus der organischen Phase durch fraktionierte Destillation bei vermindertem Druck 4-Nitrofluorbenzol in einer Ausbeute von 96 % der Theorie erhalten.

**Vergleichsbeispiel 1**

(Herstellung von 4-Nitrofluorbenzol nach dem Stand der Technik)

Es wurde verfahren wie in Beispiel 1, jedoch wurde an Stelle von (N,N-Dimethylimidazolidino)-tetramethylguanidiniumchlorid die gleiche molare Menge Tetrakis-(diethylamino)-phosphoniumbromid eingesetzt. 4-Nitrofluorbenzol wurde nach 6-stündiger Reaktionszeit in einer Ausbeute von 93 % der Theorie erhalten.

**Vergleichsbeispiel 2**

(Herstellung von 4-Nitrofluorbenzol gemäß dem Stand der Technik)

Es wurde verfahren wie in Beispiel 1, jedoch wurde anstatt (N,N-Dimethylimidazolidino)-tetramethylguanidiniumchlorid die gleiche molare Menge Tetraphenylphosphoniumbromid eingesetzt. 4-Nitrofluorbenzol wurde nach 6-stündiger Reaktionszeit in einer Ausbeute von 89 % der Theorie erhalten.

**Beispiel 2** (erfindungsgemäße Herstellung von 3-Chlor-4-fluorbenzonitril)

In einem 1 1 Vierhalskolben, der mit Ankerrührer, Thermometer und Rückflusskühler mit Blasenzähler ausgerüstet war, wurde 172 g 3,4-Dichlorbenzonitril, 200 g Dimethylsulfoxid, 69,6 g Kaliumfluorid und 3,95 g N-(N,N-Dimethylimidazolidino)-tris-(diethylamino)-phosphazeniumchlorid vorgelegt. Anschließend wurde unter Rühren auf 170°C

erhitzt und diese Temperatur für 6 Stunden beibehalten. Danach wurde auf Raumtemperatur abgekühlt, dem Reaktionsgemisch im Volumenverhältnis 1:1 Wasser zugefügt und das ausfallende 3-Chlor-4-fluorbenzonitril durch Filtration, Waschen und Trocknen isoliert. 3-Chlor-4-fluorbenzonitril wurde in einer Ausbeute von 92 % der Theorie erhalten.

**Vergleichsbeispiel 3**

(Herstellung von 3-Chlor-4-fluorbenzonitril gemäß dem Stand der Technik)

Es wurde verfahren wie in Beispiel 2, jedoch wurde als Katalysator eine entsprechende molare Menge Tetraphenylphosphoniumbromid eingesetzt. 3-Chlor-4-fluorbenzonitril wurde in einer Ausbeute von 81 % der Theorie erhalten.

**Beispiel 3**

(erfindungsgemäße Herstellung von 4-Fluorbenzonitril)

In einem 1 1-Vierhalskolben, der mit Ankerrührer, Thermometer und Rückflußkühler mit Blasenzähler ausgerüstet war, wurden 200 g 4-Chlorbenzonitril, 101,4 g Kaliumfluorid, 25 g Dimethylsulfoxid und 5,60 g (N,N-Dimethylimidazolidino)-tetramethylguanidiniumchlorid vorgelegt. Anschließend wurde unter Rühren auf 180°C erhitzt und diese Temperatur für 16 Stunden beibehalten. Danach wurde auf Raumtemperatur abgekühlt, dem Reaktionsgemisch im Volumenverhältnis 1:1 Wasser zugefügt und mit Diethylether extrahiert. Nach dem Waschen, Einengen und Trocknen der abgetrennten organischen Phase wurde 4-Fluorbenzonitril in einer Ausbeute von 75 % isoliert.

**Vergleichsbeispiel 4**

(Herstellung von 4-Fluorbenzonitril gemäß dem Stand der Technik)

In einem 250 ml-Vierhalskolben, der mit Ankerrührer, Thermometer und Rückflusskühler mit Blasenzähler ausgerüstet war, wurden 25,3 g Kaliumfluorid in 70 ml Sulfolan vorgelegt und 1 Stunde bei 100°C gerührt. Dann entfernte man den Rückflusskühler, setzte stattdessen eine Destillationsbrücke auf und destillierte bei vermindertem Druck 20 ml Sulfolan ab. Nunmehr wurde die Apparatur mit Stickstoff beaufschlagt, wieder der Rückflusskühler aufgesetzt und 50 g 4-Chlorbenzonitril und 1,52 g Tetraphenylphosphoniumbromid hinzugefügt. Anschließend wurde unter Rühren auf 180°C erhitzt und diese Temperatur für 6 Stunden beibehalten. Mittels gaschromatographischer Analyse wurde dann festgestellt, dass sich nur 2 % des eingesetzten 4-Chlorbenzonitrils zu 4-Fluorbenzonitril umgesetzt hatten.

**Beispiel 4**

(Erfindungsgemäße Herstellung von 2,4-Difluorbenzoesäurefluorid)

In einem 1 1-Vierhalskolben, der mit Ankerrührer, Thermometer und Rückflusskühler mit Blasenzähler ausgerüstet war, wurden 100 g 2,4-Dichlorbenzoylchlorid, 100 g N,N-Dimethylimidazolidin-2-on, 94,3 g Kaliumfluorid und 1,78 g (N,N-Dimethylimidazolidino)-tetramethylguanidiniumchlorid vorgelegt. Anschließend wurde unter Rühren auf 180°C erhitzt und diese Temperatur 24 Stunden beibehalten.
Danach wurde auf Raumtemperatur abgekühlt, dem Reaktionsgemisch im Volumenverhältnis 1:1 Dichlormethan zugefügt und filtriert. Aus dem Filtrat wurde das Lösungsmittel abgezogen und der Rest fraktioniert destilliert. Man erhielt 2,4-Difluorbenzoylfluorid in 75 %iger Ausbeute.

**Vergleichsbeispiel 5**

(Herstellung von 2,4-Difluorbenzolsäurefluorid gemäß dem Stand der Technik)

In einem Autoklaven wurden 200 g 2,4-Chlorbenzoylchlorid und 200 g Sulfolan vorgelegt und für 9 Stunden unter Rühren auf 200°C erhitzt. Dann ließ man auf Raumtemperatur abkühlen, entspannte und destillierte das Produkt direkt aus der Reaktionsmischung heraus. So wurde 2,4-Difluorbenzoylfluorid in 35 %iger Ausbeute isoliert.

**Beispiel 5**

(erfindungsgemäße Herstellung von Tetrafluorbenzotrifluorid)

a) In einem 1 1 Autoklaven wurden 400 g Tetrachlorbenzotrifluorid, 212 g Kaliumfluorid, 5 g (N,N-Dimethylimidazolidino)-tetramethylguanidiniumchlorid und 2 g Dichlormethan vorgelegt und unter Rühren für 8 Stunden auf 200°C erhitzt. Dann wurde auf Raumtemperatur abgekühlt, die ausgefallenen Salze abfiltriert und das Filtrat gaschromatographisch analysiert. Die Analysenergebnisse sind aus Tabelle 1 ersichtlich.

b) Das Filtrat aus dem Teilschritt a) wurde zusammen mit 6,3 g (N,N-Dimethylimidazolidino)-tetramethylguanidiniumchlorid und 193,5 g Kaliumfluorid in einem 1 1 Autoklaven vorgelegt und für 32 Stunden auf 200°C erhitzt. Danach wurde auf Raumtemperatur abgekühlt, der Autoklav entspannt und die erhaltenen teilweise und vollständig fluorierten Benzotrifluoride aus dem Reaktionsgemisch durch Destillation entfernt. Das dabei erhaltene Destillat wurde gaschromatografisch untersucht. Die Ergebnisse sind aus Tabelle 1 ersichtlich. Tetrafluorbenzotrifluorid wurde aus dem Destillat durch fraktionierte Destillation in gereinigter Form erhalten.

**Beispiel 6** (erfindungsgemäße Herstellung von Tetrafluorbenzotrifluorid)

Es wurde verfahren wie in Beispiel 5a, jedoch wurden als Katalysator 6,6 g N-(N,N-Dimethylimidazolidino)-tris-(diethylamino)-phosphazeniumchlorid und anstelle von Dichlormethan 28 g Sulfolan eingesetzt. Außerdem wurde in Teilschritt b) nur 24 Stunden lang auf 200°C erhitzt. Die Analysenergebnise nach Durchführung der Schritte a) und b) sind aus Tabelle 1 ersichtlich.

**Beispiel 7** (erfindungsgemäße Herstellung von Tetrafluorbenzotrifluorid)

Es wurde gearbeitet wie in Beispiel 5a beschrieben, jedoch wurden als Katalysator 8,24 g Diethylaminobis-(tetramethylguanidino)-sulfoniumbromid eingesetzt und in Schritt b) nur 24 Stunden auf 200°C erhitzt. Die nach Durchführung der Teilschritte a) und b) erhaltenen Analysenergebnisse sind aus Tabelle 1 ersichtlich.

**Vergleichsbeispiel 6**

(Herstellung von Tetrafluorbenzotrifluorid gemäß dem Stand der Technik)

Es wurde verfahren wie in Beispiel 5a beschrieben, jedoch wurden als Katalysator 8,38 g Tetraphenylphosphoniumbromid eingesetzt und in Teilschritt a) 28 Stunden auf 200°C erhitzt. Die nach Durchführung der Teilschritte a) und b) erhaltenen Analysenergebnisse sind aus Tabelle 1 ersichtlich.

**Tabelle 1** Gehalte an halogenierten Benzotrifluoriden (BTF) Angaben in %

| | **Tetrachlor-BTF** | **Trichlor-monofluor-BTF** | **Dichlor-difluor- BTF** | **Monochlor-trifluor- BTF** | **Tetrafluor BTF** |
|---|---|---|---|---|---|
| nach Teilschritt a) | | | | | |
| Beispiel 5 | 0 | 21 | 66 | 0 | 0 |
| Beispiel 6 | 0 | 5 | 70 | 24 | 1 |
| Beispiel 7 | 4 | 51 | 44 | 1 | 0 |
| Vergleichsbeispiel 6 | 19 | 62 | 19 | 0 | 0 |
| nach Teilschritt b) | | | | | |
| Beispiel 5 | 0 | 0 | 0 | 17 | 84 |

(fortgesetzt)

| | | | | | |
|---|---|---|---|---|---|
| nach Teilschritt b) | | | | | |
| Beispiel 6 | 0 | 0 | 0 | 33 | 67 |
| Beispiel 7 | 0 | 0 | 0 | 35 | 65 |
| Vergleichsbeispiel 6 | 0 | 14 | 78 | 6 | 1 |

**Beispiel 8** (erfindungsgemäße Herstellung von N-(N,N-Dimethylimidazolidino)-tris-(diethylaminophosphonium)-chlorid, Formel (VI))

In einem 4 1-Dreihalskolben mit Ankerrührer, Tropftrichter und Gaseinleitungsrohr wurden unter Inertgasatmosphäre 282,9 g Phosphorpentachlorid in 1000 ml Dichlormethan vorgelegt und bei -30°C 730 g Diethylamin portionsweise zugegeben. Durch entsprechende Kühlung wurde dafür gesorgt, dass die Temperatur -15°C nicht überstieg. Nach beendeter Zugabe liess man sich das Reaktionsgemisch auf Raumtemperatur erwärmen und rührte dann noch 2 Stunden nach. Anschließend wurden bei 0°C 30 g Ammoniak eingeleitet, wieder auf Raumtemperatur anwärmen lassen und 2 Stunden bei Raumtemperatur nachgerührt. Danach wurden alle flüchtigen Bestandteile des Reaktionsgemisches im Vakuum abgezogen und der Rückstand in einem Gemisch aus 350 ml Wasser und 550 g 40 gew.-%ige wässriger Natronlauge-Lösung gelöst. Nach einstündigem Rühren bei Raumtemperatur wurden Ammoniak, Diethylamin und Wasser durch Destillation abgetrennt und ein Rückstand erhalten, der im wesentlichen $(Ethyl_2N)_3P\text{-}NH_2^+Cl^-$ und Natriumchlorid enthielt. Es wurde mit 2800 ml 50 gew.-%ige wässriger Natronlauge versetzt und $(Ethyl_2N)_3P{=}NH$ durch Extraktion mit Toluol in 85 %iger Ausbeute erhalten.

Dieses (131 g) wurde portionsweise bei -10 bis -20°C zu einer Lösung aus 42,2 g 2-Chlor-1,3-dimethylimidazoliniumchlorid in 250 ml Dichlormethan gegeben. Dann wurde 4 Stunden bei 0°C und 2 Stunden bei Raumtemperatur gerührt. Anschließend wurde das Lösungsmittel im Vakuum entfernt, der Rückstand in 300 ml Methanol suspendiert und bei -20°C 18,4 g Kaliummethylat gelöst in 100 ml Methanol versetzt. Das Reaktionsgemisch wurde sich auf Raumtemperatur erwärmen lassen. filtriert und aus dem Filtrat das Lösungsmittel abgezogen. Man erhielt so N-(N,N-Dimethylimidazolidino)-tris-(diethylaminophosphonium)-chlorid in 93 %iger Reinheit.
Schmelzpunkt: 64 bis 65°C.
$^1$H-NMR (200 MHz, $CDCl_3$): $\delta$ = 0.69 (t, $^3J_{H\text{-}H}$ = 6.9H, 18H, $CH_3CH_2N$), 2.49 (s, <u>6H</u>, $CH_3N$), 2.65 (dq, $^3J_{H\text{-}H}$ = 6.9Hz, $^3J_{H\text{-}H}$ = 10.7Hz, 12H, $CH_2CH_2$) $^{31}$P-NMR (80MHz, entkoppelt): $\delta$ = 19.1 (s)
$^{13}$C-NMR (50.3MHz, $CDCl_3$): $\delta$ = 13.2 (s, $CH_3$, $CH_3CH_2N$), 33.3 (s, $CH_3$, $CH_3N$), 39.4 (s, $CH_3$, $CH_3CH_2N$), 47.3 (s, $CH_2$, $CH_2CH_2$), 155.6 (d, C=N, $^2J_{C\text{-}P}$ = 25.1Hz).

**Beispiel 9**

(Erfindungsgemäße Herstellung von Diethylaminobis-(tetramethylguanidino)-sulfoniumbromid, Formel (VII))

Unter Inertgasatmosphäre wurden bei -35°C zu einer Lösung von 8,85 g Bisdiethylaminosulfid in 40 ml Dichlormethan 7,5 g Brom zugetropft. Nach der Zugabe rührte man noch 30 Minuten bei -35°C nach und gab dann 11,5 g Tetramethylguanidin tropfenweise hinzu. Nun ließ man auf Raumtemperatur erwärmen, rührte 1 Stunde nach und kühlte dann auf 0°C ab. Bei dieser Temperatur wurden 2,8 g Natriumethylat (gelöst in 50 ml Methanol) hinzugegeben und die Mischung anschließend auf Raumtemperatur kommen gelassen. Methanol wurde im Vakuum bei 20 bis 100°C abgezogen und der Rückstand mit Pentan gewaschen. Man erhielt 17,6 g einer 4:1-Mischung des Diethylaminobis-(tetramethylguanidino)-sulfoniumbromid mit Bis-diethylamino-(tetramethylguanidino)-sulfoniumbromid. Durch Umkristallisation aus Azeton/Diethylether wurden 8,5 g (41,3 % der Theorie) Diethylaminobis-(tetramethylguanidino)-sulfoniumbromid in reiner Form erhalten. Schmelzpunkt: 117 bis 119°C
$^{13}$C-NMR (50.3 MHz, $CDCl_3$): $\delta$ = 14.5 ($CH_3$, $CH_3CH_2N$), 41.1 ($CH_3$, $CH_3N$), 41.7 ($CH_2$, $CH_3CH_2N$).

**Beispiel 10**

(Erfindungsgemäße Herstellung von Tris-(tetramethylguanidino)-sulfoniumchlorid)

Bei -78°C wurden unter Inertgasatmosphäre 10 g Schwefeldichlorid in 100 ml Dichlormethan vorgelegt und 3,4 g Chlorgas einkondensiert. Anschließend gab man 69,0 g Tetramethylguanidin hinzu und ließ sich das Gemisch langsam auf Raumtemperatur erwärmen. Das Lösungsmittel wurde im Vakuum entfernt und der Rückstand auf 0°C abgekühlt. Bei dieser Temperatur gab man 5,8 g Natriummethylat gelöst in 40 ml Methanol hinzu und ließ die Mischung

anschließend sich auf Raumtemperatur erwärmen. Das Methanol wurde im Vakuum abgezogen. Man erhielt so Tris-(tetramethylguanidino)-sulfoniumchlorid in 97 %iger Ausbeute und 96,5 %iger Reinheit.
Schmelzpunkt: 115 bis 116°C
$^1$H-NMR (200MHz, $CDCl_3$): $\delta$ = 2.83 (s, 18H, $CH_3N$)
$^{13}$C-NMR (50.3 MHz, $CDCl_3$): $\delta$ = 40.9 ($CH_3$, $CH_3N$), 165.0 (C=N).

**Beispiel 11**

(Erfindungsgemäße Herstellung von Diethylaminobis-[tris-(diethylamino)-phosphazenyl]-sulfoniumbromid, Formel VIII))

Unter Inertgasatmosphäre wurden bei -35°C zu einer Lösung von 5,7 g Bisdiethylaminosulfid in 25 ml Dichlormethan 5,0 g Brom zugetropft. Nach beendeter Zugabe rührte an noch 30 Minuten bei dieser Temperatur nach und gab dann 7,2 g Tris-(diethylamino)-phosphazen hinzu. Dann ließ man auf Raumtemperatur erwärmen, rührte eine Stunde nach und kühlte wiederum auf 0°C ab. Bei dieser Temperatur gab man 1,8 g Natriummethylat gelöst in 40 ml Methanol hinzu und ließ die Mischung anschließend sich auf Raumtemperatur erwärmen. Das Methanol wurde im Vakuum abgezogen und der Rückstand zweimal mit Pentan gewaschen. Man erhielt eine 4:1-Mischung des Diethylamino-bis-[tris-(diethylamino)-phosphazenyl]-sulfoniumbromid mit Bis-(dimethylamino)-tris-(diethylamino)-phosphazenylsulfoniumbromid.
$^{31}$P-NMR (80MHz, entkoppelt): 38.4 (s, 20 %), 32.7 (s, 80 %).

**Patentansprüche**

1. Verfahren zur Herstellung von kernfluorierten Aromaten durch Umsetzung einer aromatischen Verbindung, die am Kern mit gegen Fluor austauschbarem Halogen substituiert ist, mit einem Fluorid, **dadurch gekennzeichnet, dass** man es bei 40 bis 260°C und in Gegenwart mindestens einer Verbindung der Formel (I) durchführt

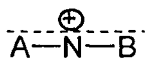 An$^{\ominus}$ (I),

in der

A für einen Rest der Formeln (II) oder (III)

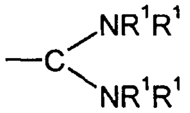

(II)

$-P(NR^1R^1)_3$

(III)

und
B unabhängig von A für einen Rest der Formeln (II), (III), (IV) oder (IVa)

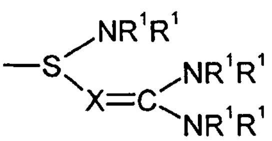

(IV)

$-S(NR^1R^1)_2$ (IVa)

stehen,
wobei
die einzelnen $R^1$ gleich oder verschieden sind und jeweils für geradkettiges oder verzweigtes $C_1$-$C_{10}$-Alkyl, geradkettiges oder verzweigtes $C_2$-$C_{10}$-Alkylen oder $C_6$-$C_{12}$-Aryl stehen,
wobei eine oder mehrere $NR^1R^1$-Gruppen auch für einen 3- bis 5-gliedrigen, gesättigten oder ungesättigten Ring stehen können, der aus einem Stickstoffatom und sonst C-Atomen gebildet wird,
wobei die Formel (II) und die

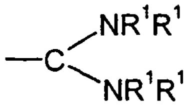

-Gruppe in Formel (IV) auch für den Rest eines gesättigten oder ungesättigten 4- bis 8-gliedrigen Rings stehen kann, der zwei Stickstoffatome und sonst Kohlenstoffatome enthält,
X für Stickstoff oder Phosphor steht und
$An^{\ominus}$ ein Äquivalent eines Anions bedeutet.

2. Verfahren nach Anspruch 1, **dadurch gekennzeichnet, dass** die Reste $R^1$ für Methyl, Ethyl, Propyl oder Butyl oder eine $NR^1R^1$-Gruppe für einen 5- bis 7-gliedrigen gesättigten oder ungesättigten Ring, der aus einem Stickstoffatom und sonst C-Atomen gebildet wird
oder
die Formel (II) oder die

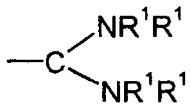

-Gruppe in Formel (IV) für einen gesättigten 5- bis 7-gliedrigen Ring, der 2 Stickstoffatome und sonst Kohlenstoffatome enthält,

X für Stickstoff und
$An^{\ominus}$ für Chlorid, Bromid, $(CH_3)_3SiF_2^{\ominus}$, $HF_2^{\ominus}$, $H_2F_2^{\ominus}$, Tetrafluoroborat, Hexafluorophosphat, Carbonat oder Sulfat stehen.

3. Verfahren nach Ansprüchen 1 und 2, **dadurch gekennzeichnet, dass** man kernfluorierte Aromaten der Formel (X) herstellt

$$R^2_xArF_wCl_{(y-w)}R_z^3 \qquad (X),$$

in der

$R^2$ unabhängig voneinander für F, Cl, Br, $NO_2$ CN, $CF_3$, $CCl_3$, CHO, $OCF_3$, $SCF_3$, $COR^4$, $COOR^4$, COY oder $SO_2Y$ mit $R^4$ = $C_1$-$C_{10}$-Alkyl und Y = F, Cl, Br oder $CF_3$ steht,
$R^3$ unabhängig voneinander für Wasserstoff oder einen geradkettigen oder verzweigten $C_1$-$C_{10}$-Alkyl- oder $C_1$-$C_{10}$-Alkoxyrest steht,
x eine ganze Zahl von 1 bis 3 bedeutet,
Ar für einen aromatischen oder heteroaromatischen Rest mit insgesamt 6 bis 10 Ringatomen steht, wobei es sich bei den Ringatomen nur um Kohlenstoffatome handeln kann, aber auch um Kohlenstoffatome plus 1 bis 3 Heteroatome aus der Gruppe Stickstoff, Sauerstoff und Schwefel, steht,
w eine ganze Zahl von1 bis y bedeutet,
y eine ganze Zahl von 1 bis 5 bedeutet und
z Null oder eine ganze Zahl von 1 bis 5 bedeutet, wobei gilt: x+y+z = Zahl aller substituierbaren Valenzen am Rest Ar

und dazu als aromatische Verbindungen, die mit gegen Fluor austauschbarem Halogen substituiert sind, solche einsetzt, die der Formel (XI) entsprechen

$$R^2_xArCl_yR^3_z \qquad (XI),$$

in der die verwendeten Symbole die bei Formel (X) angegebene Bedeutung haben.

**4.** Verfahren nach Anspruch 3, **dadurch gekennzeichnet, dass** in den Formeln (X) und (XI)

$R^2$ unabhängig voneinander für Cl, $NO_2$, CN, $CF_3$, COCl oder CHO,
$R^3$ unabhängig voneinander für Wasserstoff, Methyl, Ethyl, Methoxy oder Ethoxy,
Ar für einen Phenyl- oder Pyridylrest,
x für 1 oder 2,
w für 1 oder 2,
y für eine ganze Zahl von 1 bis 4 und
z für Null oder 1 stehen.

**5.** Verfahren nach Ansprüchen 1 bis 4, **dadurch gekennzeichnet, dass** bezogen auf 1 Mol gegen Fluor auszutauschendes Halogen, das an den Kern einer aromatischen Verbindung gebunden ist 0,001 bis 0,5 Mol einer oder mehrerer Verbindungen der Formel (I) und 0,8 bis 2 Äquivalente eines oder mehrerer Fluoride eingesetzt werden.

**6.** Verfahren nach Ansprüchen 1 bis 5, **dadurch gekennzeichnet, dass** man es in Gegenwart dipolar aprotischer und/oder unpolar aprotischer Lösungsmittel durchführt.

**7.** Verfahren nach Ansprüchen 1 bis 5, **dadurch gekennzeichnet, dass** man bei der Umsetzung von polychlorierten Benzotrifluoriden zu fluorierten und chlorierten Benzotrifluoriden und/oder zu polyfluorierten Benzotrifluoriden kann auf ein Lösungsmittel verzichtet.

**8.** Verfahren zur Herstellung von Verbindungen der Formel (I) (wie in Anspruch 1 angegeben), **dadurch gekennzeichnet, dass** man eine Verbindung der Formel

$$[A\text{-}An']^{\oplus} \qquad An^{\ominus} \qquad (XII),$$

in der

A die bei Formel (I) angegebene Bedeutung hat oder der Formel (IVa) entspricht,
An für Chlor oder Brom und
$An^{\ominus}$ für ein Äquivalent eines Anions steht,

mit einer Verbindung der Formel (XIII)

$$HN{=}A' \qquad (XIII),$$

in der

A' hinsichtlich der Anordnung der Atome die bei Formel (I) für A angegebene Bedeutung hat, aber 2-bindig ist

umsetzt und eine Base zufügt.

**9.** Verfahren nach Anspruch 8, **dadurch gekennzeichnet, dass** man als Basen Alkoholate, tertiäre Amine oder einen Überschuss an einer Verbindung der Formel (XIII) in Mengen von 1 bis 1,2 Äquivalenten, bezogen auf 1 Mol der Verbindung der Formel (XII) einsetzt.

**10.** Verfahren nach Ansprüchen 8 und 9, **dadurch gekennzeichnet, dass** man es bei -80°C bis +70°C und unter Einsatz von 0,8 bis 3 Mol einer Verbindung der Formel (XIII), bezogen auf die Verbindung der Formel (XII), durchführt und die Base bei -50 bis +40°C zugibt.

**11.** Verbindungen der Formel (I)

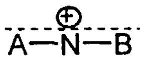 An⊖ (I),

in der

A für einen Rest der Formeln (II) oder (III)

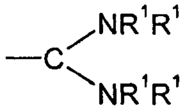

(II)

$-P(NR^1R^1)_3$

(III)

und
B unabhängig von A für einen Rest der Formeln (IV) oder (IVa)

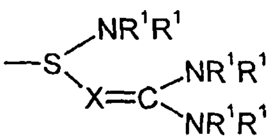

(IV)

$-S(NR^1R^1)_2$ (IVa)

stehen,
wobei
die einzelnen $R^1$ gleich oder verschieden sind und jeweils für geradkettiges oder verzweigtes $C_1$-$C_{10}$-Alkyl, geradkettiges oder verzweigtes $C_2$-$C_{10}$-Alkylen oder $C_6$-$C_{12}$-Aryl stehen,
wobei eine oder mehrere $NR^1R^1$-Gruppen auch für einen 3- bis 5-gliedrigen, gesättigten oder ungesättigten Ring stehen können, der aus einem Stickstoffatom und sonst C-Atomen gebildet wird,
wobei die Formel (II) und die

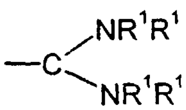

-Gruppe in Formel (IV) auch für den Rest eines gesättigten oder ungesättigten 4- bis 8-gliedrigen Rings stehen kann, der zwei Stickstoffatome und sonst Kohlenstoffatome enthält,
X für Stickstoff oder Phosphor steht und
An⊖ ein Äquivalent eines Anions bedeutet.

**12.** Diethylamino-bis-(tetramethylguanidino)-sulfoniumbromid.

**13.** Diethylamino-bis-[tris-(diethylamino)-phosphazenyl]-sulfoniumbromid.

**14.** Verbindung der Formel (VI)

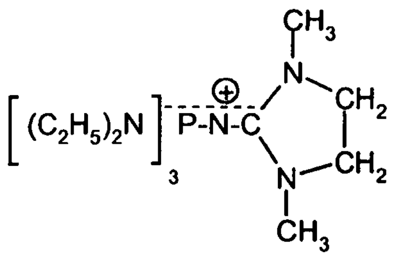

$Cl^{\ominus}$ (VI)

## Claims

**1.** Process for preparing nuclear-fluorinated aromatics by reacting, with a fluoride, an aromatic compound which is substituted at the nucleus with halogen exchangeable for fluorine which is **characterized in that** the process is carried out at 40 to 260°C and in the presence of at least one compound of the formula (I),

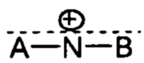

$An^{\ominus}$ (I),

where

A is a radical of the formula (II) or (III)

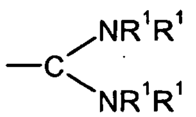

(II)

$-P(NR^1R^1)_3$

(III)

and
B independently of A is a radical of the formula (II), (III), (IV) or (IVa)

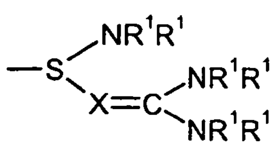

(IV)

$-S(NR^1R^1)_2$ (IVa),

where
the individual $R^1$ are identical or different and are each unbranched or branched $C_1$-$C_{10}$ alkyl, unbranched or branched $C_2$-$C_{10}$ alkylene or $C_6$-$C_{12}$ aryl,
where one or more $NR^1R^1$ groups can also be a 3- to 5-membered saturated or unsaturated ring which is formed from a nitrogen atom and the rest carbon atoms,
where the formula (II) and the

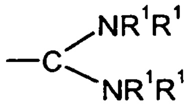

group in formula (IV) can also be the radical of a saturated or unsaturated 4- to 8-membered ring which contains two nitrogen atoms and the remainder carbon atoms,

X is nitrogen or phosphorus and

$An^e$ is one equivalent of an anion.

2. Process according to Claim 1, **characterized in that** the radicals $R^1$ are methyl, ethyl, propyl or butyl, or an $NR^1R^1$ group is a 5- to 7-membered saturated or unsaturated ring which is formed from one nitrogen atom and the remainder carbon atoms
or
the formula (II) or the

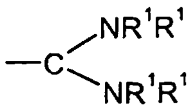

group in formula (IV) is a saturated 5- to 7-membered ring which contains 2 nitrogen atoms and the remainder carbon atoms,

X is nitrogen and

$An^\ominus$ is chloride, bromide, $(CH_3)_3SiF_2^\ominus$, $HF_2^\ominus$, $H_2F_2^\ominus$, tetrafluoroborate, hexafluorophosphate, carbonate or sulphate.

3. Process according to Claims 1 and 2, **characterized in that** nuclear-fluorinated aromatics of the formula (X) are prepared

$$R^2_xArF_wCl_{(y-w)}R_z^3 \qquad (X),$$

where

$R^2$ independently of one another is F, Cl, Br, $NO_2$, CN, $CF_3$, $CCl_3$, CHO, $OCF_3$, $SCF_3$, $COR^4$, $COOR^4$, COY or $SO_2Y$ where $R^4$ = $C_1$-$C_{10}$ alkyl and Y = F, Cl, Br or $CF_3$,

$R^3$ independently of one another is hydrogen or an unbranched or branched $C_1$-$C_{10}$ alkyl or $C_1$-$C_{10}$ alkoxy radical,

x is an integer from 1 to 3,

Ar is an aromatic or heteroaromatic radical having a total of 6 to 10 ring atoms, where the ring atoms can be only carbon atoms, or alternatively carbon atoms plus 1 to 3 heteroatoms selected from the group consisting of nitrogen, oxygen and sulphur,

w is an integer from 1 to y,

y is an integer from 1 to 5 and

z is zero or an integer from 1 to 5, where x+y+z = the number of all substitutable valencies on the radical Ar

and the aromatic compounds which are substituted with halogen exchangeable for fluorine used are those which correspond to the formula (XI)

$$R^2_xArCl_yR^3_z \qquad (XI),$$

where the symbols used have the meaning specified under formula (X).

4. Process according to Claim 3, **characterized in that** in the formulae (X) and (XI)

$R^2$ independently of one another is Cl, $NO_2$, CN, $CF_3$, COCl or CHO,
$R^3$ independently of one another is hydrogen, methyl, ethyl, methoxy or ethoxy,
Ar is a phenyl or pyridyl radical,
x is 1 or 2,
w is 1 or 2,
y is an integer from 1 to 4 and
z is zero or 1.

5. Process according to Claims 1 to 4, **characterized in that**, based on 1 mol of halogen which is bound to the nucleus of an aromatic compound and is to be exchanged for fluorine, 0.001 to 0.5 mol of one or more compounds of the formula (I) and 0.8 to 2 equivalents of one or more fluorides are used.

6. Process according to Claims 1 to 5, **characterized in that** it is carried out in the presence of dipolar aprotic solvents and/or nonpolar aprotic solvents.

7. Process according to Claims 1 to 5, **characterized in that** a solvent is not necessary in the conversion of polychlorinated benzotrifluorides to fluorinated and chlorinated benzotrifluorides and/or to polyfluorinated benzotrifluorides.

8. Process for preparing compounds of the formula (I) (as specified in Claim 1), **characterized in that** a compound of the formula

$$[A\text{-}An']^{\oplus} \quad An^{\ominus} \qquad (XII),$$

where

A has the meaning specified under formula (I) or corresponds to the formula (IVa),
An' is chlorine or bromine and
$An^{\ominus}$ is one equivalent of an anion,

is reacted with a compound of the formula (XIII)

$$HN{=}A' \qquad (XIII),$$

where

A' with respect to the arrangement of the atoms has the meaning specified for A under formula (I), but is double-bonded

and a base is added.

9. Process according to Claim 8, **characterized in that** the bases used are alkoxides, tertiary amines or an excess of a compound of the formula (XIII) in amounts of 1 to 1.2 equivalents, based on 1 mol of the compound of the formula (XII).

10. Process according to Claims 8 and 9, **characterized in that** it is carried out at -80°C to +70°C using 0.8 to 3 mol of a compound of the formula (XIII), based on the compound of the formula (XII), and the base is added at -50 to +40°C.

11. Compounds of the formula (I)

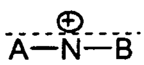 $An^{\ominus}$ (I),

where

A is a radical of the formula (II) or (III)

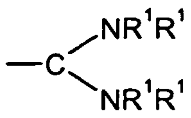

(II)

$-P(NR^1R^1)_3$

(III)

and

B independently of A is a radical of the formula (IV) or (IVa)

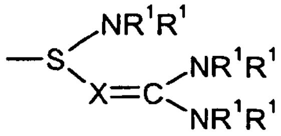

(IV)

$-S(NR^1R^1)_2$ (IVa),

where

the individual $R^1$ are identical or different and in each case are an unbranched or branched $C_1$-$C_{10}$ alkyl, unbranched or branched $C_2$-$C_{10}$ alkylene or $C_6$-$C_{12}$ aryl,

where one or more $NR^1R^1$ groups can also be a 3- to 5-membered, saturated or unsaturated ring which is formed from one nitrogen atom and the remainder carbon atoms,

where the formula (II) and the

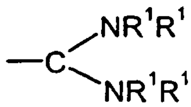

group in formula (IV) can also be the radical of a saturated or unsaturated 4- to 8-membered ring which contains two nitrogen atoms and the remainder carbon atoms,

X is nitrogen or phosphorus and

$An^{\ominus}$ is one equivalent of an anion.

**12.** Diethylaminobis(tetramethylguanidino)sulphonium bromide.

**13.** Diethylaminobis[tris(diethylamino)phosphazenyl]sulphonium bromide.

**14.** Compounds of the formula (VI)

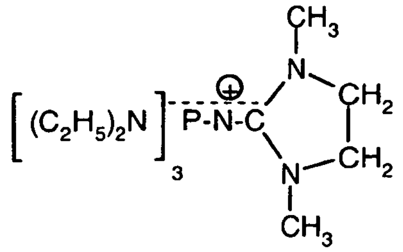

$Cl^{\ominus}$ (VI)

## Revendications

**1.** Procédé de préparation de composés aromatiques fluorés sur le noyau par réaction d'un composé aromatique, qui est substitué sur le noyau par un halogène échangeable contre un fluore, avec un fluorure, **caractérisé en ce qu'**il est réalisé entre 40 et 260°C et en présence d'au moins un composé de formule (I)

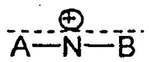 $An^{\ominus}$ (I),

dans laquelle

A représente un radical de formules (II) ou (III)

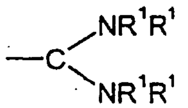

(II)

$-P(NR^1R^1)_3$

(III)

et
B, indépendamment de A, représente un radical de formules (II), (III), (IV) ou (IVa)

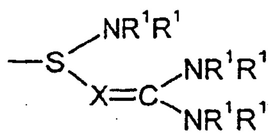

(IV)

$-S(NR^1R^1)_2$ (IVa)

dans lesquelles
les groupes $R^1$ individuels sont identiques ou différents et représentent chacun un groupe alkyle en $C_1$-$C_{10}$ linéaire ou ramifié, un groupe alkylène en $C_2$-$C_{10}$ linéaire ou ramifié ou un groupe aryle en $C_6$-$C_{12}$,
où un ou plusieurs groupes $NR^1R^1$ peuvent également représenter un noyau saturé ou insaturé à 3-5 chaînons, qui est formé d'un atome d'azote et par ailleurs d'atomes de carbone,
où la formule (II) et le groupe

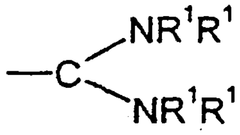

dans la formule (IV) peuvent également représenter le radical d'un noyau saturé ou insaturé, à 4-8 chaînons, qui renferme deux atomes d'azote et par ailleurs des atomes de carbone,
x représente un atome d'azote ou un atome de phosphore, et
$An^{\ominus}$ représente un équivalent d'un anion.

2. Procédé selon la revendication 1, **caractérisé en ce que** les radicaux $R^1$ représentent un groupe méthyle, un groupe éthyle, un groupe propyle ou un groupe butyle, ou
un groupe $NR^1R^1$ représente un noyau saturé ou insaturé, à 5-7 chaînons, qui est formé d'un atome d'azote et par ailleurs d'atomes de carbone,
ou
la formule (II) ou le groupe

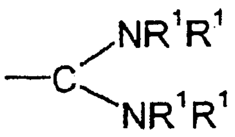

dans la formule (IV) représente un noyau saturé à 5-7 chaînons, qui renferme 2 atomes d'azote et par ailleurs des atomes de carbone,

x représente un atome d'azote, et
$An^{\ominus}$ représente un groupe chlorure, un groupe bromure, un groupe $(CH_3)_3SiF_2^{\ominus}$, un groupe $HF_2^{\ominus}$, un groupe $H_2F_2^{\ominus}$, un groupe tétrafluoroborate, un groupe hexafluorophosphate, un groupe carbonate ou un groupe sulphate.

3. Procédé selon les revendications 1 et 2, **caractérisé en ce que** l'on prépare des composés aromatiques fluorés sur le noyau de formule (X)

$$R^2_xArF_wCl_{(y-w)}R_z^3 \qquad (X),$$

dans laquelle

$R^2$ représente indépendamment un groupe F, un groupe Cl, un groupe Br, un groupe $NO_2$ un groupe CN, un groupe $CF_3$, un groupe $CCl_3$, un groupe CHO, un groupe $OCF_3$, un groupe $SCF_3$, un groupe $COR^4$, un groupe $COOR^4$ un groupe COY ou un groupe $SO_2Y$ où $R^4$ représente un groupe alkyle en $C_1$-$C_{10}$ et Y représente un groupe F, un groupe Cl, un groupe Br ou un groupe $CF_3$,
$R^3$ représente indépendamment un atome d'hydrogène ou un radical alkyle en $C_1$-$C_{10}$ ou alcoxy en $C_1$-$C_{10}$ linéaire ou ramifié,
x représente un entier de 1 à 3,
Ar représente un radical aromatique ou hétéroaromatique renfermant au total de 6 à 10 atomes du noyau, où les atomes du noyau peuvent être uniquement des atomes de carbone, mais également des atomes de carbone plus de 1 à 3 hétéroatomes parmi le groupe constitué d'un atome d'azote, d'un atome d'oxygène et d'un atome de soufre,
w représente un entier de 1 à y,
y représente un entier de 1 à 5, et
z vaut zéro ou représente un entier de 1 à 5, où x+y+z = le nombre de toutes les valences substituables sur le radical Ar,

et, à cette fin, en tant que composés aromatiques qui sont substitués par un halogène échangeable contre du fluore,

on utilise ceux qui correspondent à la formule (XI)

$$R^2{}_xArCl_yR^3{}_z \qquad (XI),$$

dans laquelle les symboles utilisés présentent la signification indiquée pour la formule (X).

4. Procédé selon la revendication 3, **caractérisé en ce que**, dans les formules (X) et (XI),

   $R^2$ représente indépendamment un groupe Cl, un groupe $NO_2$, un groupe CN, un groupe $CF_3$, un groupe COCl ou un groupe CHO,
   $R^3$ représente indépendamment un atome d'hydrogène, un groupe méthyle, un groupe éthyle, un groupe méthoxy ou un groupe éthoxy,
   Ar représente un radical phényle ou un radical pyridyle,
   x vaut 1 ou 2,
   w vaut 1 ou 2,
   y représente un entier de 1 à 4, et
   z vaut zéro ou 1.

5. Procédé selon les revendications 1 à 4, **caractérisé en ce que**, par rapport à 1 mol d'halogène à échanger contre du fluore, qui est lié au noyau d'un composé aromatique, on utilise de 0,001 à 0,5 mol d'un ou de plusieurs composés de formule (I) et de 0,8 à 2 équivalents d'un ou de plusieurs fluorures.

6. Procédé selon les revendications 1 à 5, **caractérisé en ce qu'**il est réalisé en présence de solvants aprotiques dipolaires et/ou de solvants aprotiques non polaires.

7. Procédé selon les revendications 1 à 5, **caractérisé en ce que**, lors de la réaction de benzotrifluorures polychlorés pour donner lieu à des benzotrifluorures fluorés et chlorés et/ou à des benzotrifluorures polyfluorés, on peut se passer d'un solvant.

8. Procédé de préparation de composés de formule (I) (telle qu'indiquée dans la revendication 1), **caractérisé en ce qu'**un composé de formule

$$[A\text{-}An']^{\oplus} \quad An^{\ominus} \qquad (XII),$$

dans laquelle

   A présente la signification indiquée pour la formule (I) ou correspond à la formule (IVa),
   An représente un atome de chlore ou un atome de brome, et
   $An^{\ominus}$ représente un équivalent d'un anion,

est mis à réagir avec un composé de formule (XIII)

$$HN{=}A' \qquad (XIII),$$

dans laquelle

   A' présente la signification indiquée pour A pour la formule (I) en ce qui concerne la disposition des atomes, mais est bivalent,

et une base est ajoutée.

9. Procédé selon la revendication 8, **caractérisé en ce que** l'on utilise, en tant que bases, des alcoolates, des amines tertiaires ou un excès d'un composé de formule (XIII) en quantités de 1 à 1,2 équivalents, par rapport à 1 mol du composé de formule (XII).

**10.** Procédé selon les revendications 8 et 9, **caractérisé en ce qu'**il est réalisé entre -80°C et +70°C et en utilisant de 0,8 à 3 mol d'un composé de formule (XIII), par rapport au composé de formule (XII), et la base est ajoutée entre -50 et ±40°C.

**11.** Composés de formule (I)

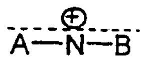 An⊖ (I),

dans laquelle

A représente un radical de formules (II) ou (III)

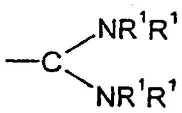

(II)

$-P(NR^1R^1)_3$

(III)

et
B, indépendamment de A, représente un radical de formules (IV) ou (IVa)

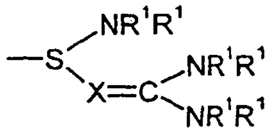

(IV)

$-S(NR^1R^1)_2$ (IVa)

dans lesquelles
les groupes $R^1$ individuels sont identiques ou différents et représentent chacun un groupe alkyle en $C_1$-$C_{10}$ linéaire ou ramifié, un groupe alkylène en $C_2$-$C_{10}$ linéaire ou ramifié ou un groupe aryle en $C_6$-$C_{12}$,
où un ou plusieurs groupes $NR^1R^1$ peuvent également représenter un noyau saturé ou insaturé à 3-5 chaînons, qui est formé d'un atome d'azote et par ailleurs d'atomes de carbone,
où la formule (II) et le groupe

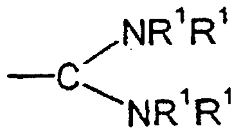

dans la formule (IV) peuvent également représenter le radical d'un noyau saturé ou insaturé, à 4-8 chaînons, qui renferme deux atomes d'azote et par ailleurs des atomes de carbone,
X représente un atome d'azote ou un atome de phosphore, et
An⊖ représente un équivalent d'un anion.

**12.** Bromure de diéthylamino-bis(tétraméthylguanidino)sulfonium.

**13.** Bromure de diéthylamino-bis[tris(diéthylamino)phosphazényl]sulfonium.

**14.** Composé de formule (VI)

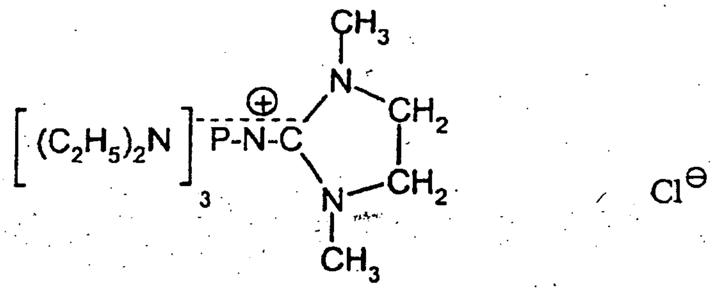

(VI)